**Europäisches Patentamt**

**European Patent Office**

**Office européen des brevets**

(19)

(11) Publication number: **0 206 779**
A1

(12) # EUROPEAN PATENT APPLICATION

(21) Application number: **86304757.7**

(22) Date of filing: **20.06.86**

(51) Int. Cl.⁴: **G 01 N 33/543**, G 01 N 33/564 // G01N33/52

(30) Priority: **21.06.85 US 747412**

(43) Date of publication of application: **30.12.86** Bulletin 86/52

(84) Designated Contracting States: **AT BE CH DE FR GB IT LI LU NL SE**

(71) Applicant: **MODERN DIAGNOSTICS, INC., 5895 Power Inn Road, Sacramento California 95824 (US)**

(72) Inventor: **Lipp, Virginia L., 2317 Elendil Lane, Davis California 95616 (US)**
Inventor: **Buck, Robert L., 3940 G Parkway, Sacramento California 95823 (US)**

(74) Representative: **Sheard, Andrew Gregory et al, Kilburn & Strode 30, John Street, London WC1N 2DD (GB)**

(54) **Detecting antinuclear antibody.**

(57) Apparatus for detecting antinuclear antibody in a biological sample, including a solid support having adhered thereto a plurality of nuclei isolated from eukaryotic cells.

EP 0 206 779 A1

0206779

- 1 -

DETECTING ANTINUCLEAR ANTIBODY

This invention relates to the detection of antinuclear antibodies in biological samples from human patients, e.g., serum samples.

The blood of human patients suffering from some medical disorders is characterized by the presence of autoantibodies, which are reactive with one or more antigens present in the nuclei of cells of the patients. It is believed that these autoantibodies are at least in part responsible for the symptoms characteristic of the diseases. Nuclear antigens against which antibodies can be generated include DNA, nuclear proteins, and nuclear polysaccharides. Medical disorders characterized by the presence of antinuclear antibodies in the blood include systemic lupus erythmatosus (SLE); mixed connective tissue disease; Sjogren's syndrome; and scleroderma.

To facilitate appropriate and timely diagnosis and treatment, it is important to detect the presence of antinuclear antibodies in serum at an early stage. Currently, such screening of serum is carried out e.g., by the indirect immunofluorescence antinuclear antibody test described, e.g., in Molden et al. (1983) Am. J. Clin. Path. 82,1, 57, involving growing cells, e.g., of the human HEp-2 cell line, on glass slides, treating the cells to increase the permeability of the cytoplasmic membrane, applying a serum sample to the slide, and detecting immune complexes between antinuclear antibodies and the nuclear antigens of the cells on the slide by indirect immunofluorescence.

In general, the invention features the detection of antinuclear antibodies in a biological sample by adhering a plurality of eukaryotic nuclei to a solid support, e.g., a 96-well ELISA plate; contacting the sample with the support under conditions sufficient to allow antinuclear antibodies in the sample to form immune complexes with nuclear antigens in the cell nuclei; and detecting the immune complexes as an indication of the presence of antinuclear antibodies in the sample.

Preferably, underlying the nuclei on the solid support is a coating, e.g., of nuclear antigens, which is substantially unreactive with antibodies to non-nuclear antigens, and which, like the nuclei, serves to bind antinuclear antibodies in the sample. It is particularly advantageous to use two different nuclear antigen-containing coatings, e.g., one prepared by rupturing the same nuclei as are used in intact form, and the second from a different source; the antigen of this second source, in addition to binding antinuclear antibody in the sample, stabilizes the other coating component by binding its extractable nuclear antigens. This combination of nuclei and coating provides a much more effective screening apparatus than either nuclei or coating alone.

In other preferred embodiments, the detection of immune complexes is carried out by means of a labelled anti-human antibody, most preferably labelled by conjugation with an enzyme which acts chemically to alter a substrate which in turn acts on a reagent to cause a colour change.

The invention permits the screening of human serum for the presence of antinuclear antibodies in a system featuring speed, simplicity, sensitivity, and capacity for automation hitherto available only for

- 3 -

detection of specific antibodies reactive with particular immobilized antigens, but not for screening of samples for the presence of antinuclear antibodies. The invention will allow the large-scale screening of serum samples to identify patients whose serum contains antinuclear antibodies and thus should be subjected to specific diagnostic tests to identify the particular autoimmune disease from which the patient is suffering, and expedite the initiation of appropriate treatment.

Other features and advantages of the invention will be apparent from the following description of the preferred embodiments thereof, and from the claims.

A particular embodiment of the ANA test of the invention is described below.

Plate Preparation

Test plates are coated with three nuclear antigen containing materials:

1) intact human cell nuclei, designated "GN";

2) nuclear antigen of ruptured human cell nuclei, designated "NS"; and

3) calf thymus antigen (containing DNA and nuclear proteins), designated "ENA."

The first step is to grow HEp-2 cells, seeded at one-third confluency, for three days in 150 $cm^2$ culture dishes. HEp-2 cells are a human oesophageal tumour cell line, available from the American Type Culture Collection, Rockville, Maryland, ATCC Accession No. CCL23. The culture dishes are placed in a fume hood and the culture media aspirated into a two-litre collection flask. Ten ml of saline are then added to each dish, and the dishes are gently rocked to wash media from the cells. The saline is then aspirated into a second two-litre collection flask.

Five ml of saline are then added to each dish and the cells scraped from the dish using a rubber policeman on a glass rod. The cells are transferred to a 50 ml polypropylene centrifuge tube with a pasteur pipet, and centrifuged five minutes at 500 xg (1500 rpm) on a Centra 7R centrifuge. The supernatant is discarded and the cell pellet completely resuspended to the original volume in phosphate buffered saline containing, per litre deionized $H_2O$, 0.4 g $NaH_2PO_4$; 6.8g $Na_2HPO_4$; and 3.0 g NaCl, adjusted to pH 8.0 with 6N NaOH or 6N HCl. For the GN component, one-tenth of the total volume of cell suspension is placed in a separate 50 ml centrifuge tube, to which are added 0.02 ml of 25% glutaraldehyde per ml of cell suspension. The suspension is mixed and allowed to stand for 30 min. at room temperature.

To the remaining nine-tenths of the cell suspension are added 10.0% Triton X-100, a detergent, in phosphate buffered saline, pH 8.0, above; the detergent solution is added in a volume one-twentieth that of the cell suspension. The suspension is vortexed for 10-15 seconds to extract cytoplasm and to completely dissolve the detergent. The cells are centrifuged for five minutes at 500 xg, the supernatant discarded, and the pellet completely resuspended by vortexing in the original volume of saline, and the cellular material again centrifuged at 500 xg.

The supernatant is then discarded and the nuclei resuspended in one-half the previous volume of nuclei buffer consisting of 1.21 g Tris and 0.38 g EDTA per litre of deionized water adjusted to pH 8.1 with 6 N

HCl. The nuclei are then sonicated for two minutes at an output of 5, 20% pulsed duty cycle on a Branson 200 sonifier. The nuclear material is then centrifuged for 10 min. at 10,000 rpm and the supernatant, NS, saved.

The GN suspension above, is mixed with one-twentieth its volume of 10.0% Triton X-100, vortexed 10-15 seconds to extract the cytoplasm, and centrifuged 5 min. at 500 xg. The supernatant is discarded and the pellet resuspended in the original GN volume of saline containing 1 mg/ml DL-Lysine, and allowed to stand for ten min. at room temperature. The nuclei are then centrifuged for 5 min. at 1500 xg (2700 rpm), the supernatant discarded, and the pellet resuspended and sonicated as above in the original GN volume of nuclei buffer, above; this suspension is the final GN, containing intact nuclei.

To prepare ENA, 100 mg of lypophilized calf thymus acetone powder (commercially available from Immunovision, Springdale, Arkansas, USA) are mixed with 5 ml of phosphate buffered saline, pH 8.0. The Calf thymus preparation contains both DNA and non-DNA nuclear antigens.

The calf thymus suspension is sonicated as described previously and centrifuged for 10 min. at 10,000 rpm; the supernatant is the final ENA preparation.

The coating mixture is then prepared by mixing together equal volumes of NS (5.0 mg protein/ml); GN (2.5 mg protein/ml); and ENA (1.0 mg protein/ml); protein is determined by the Lowry method. The acceptable concentration ranges of these components in the mixture is 0.005 to 50 mg/ml NS; 0.005 to 50 mg/ml GN; and 0.005 to 50 mg/ml ENA. One hundred and fifty microlitres of the coating mixture per well are added to microtiter plates, e.g., such as are made by Dynatech or Nunc. Particularly useful are Dynatech Remova-Wells and Nunc MicroWell modules, which are stip-wells. Wells are dried down overnight at 37$^{o}$C. The plates are then sealed and stored at 4$^{o}$C until use.

Use

The ANA plates, above, are used to screen human serum samples for the presence of antinuclear antibodies in a two-step (sequential) assay, or in a one-step (simultaneous) assay.

Two-Step Assay

Fifty microlitres of undiluted serum are added to each plate, and the plates incubated for 15-30 min. at room temperature. The undiluted serum is then aspirated off and fifty microlitres of conjugate consisting of goat antibody against human Fc IgG coupled to the enzyme horseradish peroxidase (HRP) is added to the plates; the anti-Fc antibody is commercially available. The HRP-antibody conjugate is prepared by synthesizing the g-MBS derivative of anti-Fc IgG antibody and the deacetylated SATA derivative of HRP; the two derivatives are then reacted to form the conjugate containing three HRP molecules/antibody molecule. The g-MBS derivative of anti-Fc IgG antibody is prepared as follows.

The antibody, as purchased, is in powdered form, and must be reconsituted. The antibody solution is then filtered and the antibody concentration adjusted to 5.0 mg/ml. 37.5ml of a stock solution of g-MBS in DMF (10mg/ml) is then added dropwise to 2ml of the antibody solution with stirring. After 5 minutes, another 37.5ml of g-MBS solution is added (final antibody/g-MBS molar ratio = 1:40) and the entire mixture is stirred for 5 minutes following the addition. The mixture is then passed over an equilibrated G-25 desalting column, followed by dialysis to remove free g-MBS completely. The g-MBS-derivatized antibody is then added to the SATA derivative of HRP, which is prepared as follows.

6.93mg SATA is dissolved in 200ml DMF and 119ml of the solution is added with stirring to a solution of 50mg HRP in 0.05M phosphate EDTA buffer (4.0mg/ml, pH 7.5). The resulting mixture is allowed to react for 10 minutes to yield 1 mol sulphydryl groups/mol HRP. The reaction mixture is then desalted on a Sephadex G-25 desalting column previously equilibrated with phosphate EDTA buffer and the brown peak is collected for deacetylation.

To deacetylate the SATA-HRP derivative, 100ml of a 50mM aqueous solution of hydroxylamine containing 2.5mM EDTA is added to the SATA-HRP derivative and the mixture is stirred at room temperature for 1 hour. The mixture is then desalted on a Sephadex G-25 desalting column previously equilibrated with phosphate EDTA buffer and the brown peak, containing the deacetylated SATA-HRP derivative, is collected.

14 mg of the deacetylated SATA-HRP derivative are combined with 16.5mg of the g-MBS-antibody derivative and incubated for 1 hour at room temperature while stirring to yield the HRP-antibody conjugate. The conjugate is then dialyzed overnight in a stabilized buffer solution containing 50mM Tris, pH 7.2; 10mM EDTA/Iron (III); 10% D-mannitol; and 2% sucrose. The dialyzed conjugate is then stored in lyophilized form until ready for use.

0206779

- 8 -

The conjugate is aspirated off of the plates, and the plates washed twice with wash buffer containing, per 20 litres deionized water, 24.2 g Trizma base, 176 g NaCl, 0.04 g Thimerosal, and 20ml Tween 20, adjusted to pH 8.0 with 6.0 $\underline{N}$ HCl.

The next step is to add the color reagent, containing the oxidizable colour reagent O-phenylenediamine (OPD). The colour reagent is made by dissolving a commercially available OPD tablet (0.04 g) in 5 ml deionized water containing 0.105 g citrate monohydrate adjusted to pH 4.5, while stirring, with $6\underline{N}$ NaOH. Fifty microlitres of colour reagent, containing urea peroxide, are added to each plate, and the plates incubated for 15-30 minutes at room temperature.

The reaction is then stopped by the addition of fifty microlitres of 1.0 $\underline{N}$ $H_2SO_4$, and the plates read at 490 nm in an optical density reading instrument. Absorbance is a measure of antinuclear antibody present in the serum sample.

One-Step Assay

Fifty microlitres undiluted serum are added to each plate, and the plates incubated for 15-30 minutes at room temperature. Fifty microlitres of anti-Fc/HRP conjugate are then added, and the plates incubated an additional 15-30 minutes.

The conjugate is then aspirated and the plates washed twice with wash buffer. Fifty microlitres of OPD/peroxide solution are then added, the plates incubated for 15-30 minutes, the reaction stopped with 1.0 $\underline{N}$ $H_2SO_4$, and absorbance read at 490 nm.

## Mechanism

In both the two-step and one-step procedures, the mechanism of action, and rationale for the use of various components, are the same, and can be summarized as follows.

The purpose of the nuclear antigen coating (NS together with ENA) underlying the intact cell nuclei is the elimination of background, which could otherwise be caused by the non-specific binding of antibody in the serum sample to the wells of the plate. Most of the antibodies in the serum which are not antinuclear will fail to bind to the coating, and background is thus minimized. The ENA also serves to bind certain extractable nuclear antigens of the NS.

The intact nuclei and coating, because they contain a diversity of nuclear antigens, cause the binding to the plate of virtually all antinuclear antibodies in the sample, so that sensitivity is high and the risk of false negatives is low.

When the serum is incubated with the plates, the antinuclear antibodies present bind to the plate nuclei and/or coating, and non-binding sample constituents, including non-nuclear antibodies, are washed away.

The anti-Fc antibody then specifically binds to the bound human antinuclear antibodies, labelling them with HRP, which acts on peroxide to form an active intermediate, which in turn oxidizes OPD to produce a yellow colour.

## Other Embodiments

Other embodiments are within the following claims.

For example, the nuclei of any eukaryotic cells can be used to prepare the plates. Generally, the closer the cells are to human cells phylogenetically, the better will be the sensitivity of the test, and primate, particularly human, cell nuclei are thus most preferred.

The antigen coating components used to minimize background and bind extractable antigens can also vary. Whole disrupted nuclear antigen fractions can be used, or nuclear fractions containing either predominantly nucleic acids (DNA and RNA), or predominantly proteins and glycoproteins, can be used. The coating nuclear antigen can be derived from any species.

The detection/label system can be any of a wide variety of commercially available systems. For example, if a second antibody/enzyme conjugate is used as described above, the color reagent 3, 3', 5, 5'-tetramethylbenzidine (Miles Laboratories) can be used in place of OPD. Instead of enzyme/colour reagent systems, other labels, e.g., fluorophores or radioisotopes, can be used, although colourimetric systems are preferred because of ease of automation.

0206779

- 11 -

CLAIMS

1. Apparatus for detecting antinuclear antibody in a biological sample, the apparatus comprising a solid support have adhered thereto a plurality of nuclei isolated from eukaryotic cells.

2. Apparatus as claimed in Claim 1, further comprising a coating underlying the nuclei, the coating being substantially unreactive with antibodies to non-nuclear antigens.

3. Apparatus as claimed in Claim 2, wherein the coating comprises nuclear antigens.

4. Apparatus as claimed in Claim 1, 2 or 3, wherein the nuclei are isolated from primate cells.

5. Apparatus as claimed in Claim 4, wherein the primate cells are of a human cell line.

6. Apparatus as claimed in Claim 5, wherein the human cell line is HEp-2.

7. A method of detecting the presence of antinuclear antibodies in a biological sample, the method comprising providing a solid support having adhered thereto a plurality of nuclei isolated from eukaryotic cells, contacting the sample with the support under conditions sufficient to allow antinuclear antibodies in the sample to form immune complexes with nuclear antigens of the nuclei, and

- 12 -

detecting the immune complexes as an indication of the presence of antinuclear antibodies in the sample.

8. A method as claimed in Claim 7, wherein the detecting step is carried out by contacting the solid support with an antihuman antibody conjugated with a label.

9. A method as claimed in Claim 8 wherein the antihuman antibody is an antibody to human IgG antibody or a fraction thereof.

10. A method as claimed in Claim 8, wherein the label comprises an enzyme, and the method includes contacting the support with a substrate for the enzyme capable of producing a visually detectable change when acted on by the enzyme.

11. A method as claimed in Claim 10 wherein the substrate, when acted on by the enzyme, is chemically altered, and the method comprises contacting the solid support with a reagent which undergoes a colour change in the presence of the chemically altered substrate.

## European Patent Office

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 094 603  (AMERICAN HOECHST CORP.) <br> * page 2, paragraph 3; page 3, line 12 - page 4, line 6; page 6, lines 26/27; page 7, lines 6, 24-26; page 8, paragraphs 1-3; page 10, lines 4-25 * | 1,4-11 | G 01 N  33/543 <br> G 01 N  33/564// <br> G 01 N  33/52 |
| | --- | | |
| A | WO-A-8 800 877  (ICL SCIENTIFIC) <br> * page 3, paragraph 2, page 3, line 26 - page 4, line 21 * | 1,4-11 | |
| | --- | | |
| A | FR-A-2 287 043  (BEHRINGWERKE AG) <br> * page 1, lines 3-7, 28 - page 2, line 4 * | 1,4,5 | |
| | --- | | **TECHNICAL FIELDS SEARCHED (Int. Cl.4)** |
| A | US-A-4 499 183  (D.J. SUJANSKY et al.) <br> * column 3, paragraph 3 * | 1,4,5 | G 01 N  33/00 |
| | --- | | |
| A | US-A-3 997 657  (B.F. DZIOBKOWSKI et al.) | | |
| | ----- | | |

The present search report has been drawn up for all claims

| Place of search | Date of completion of the search | Examiner |
|---|---|---|
| BERLIN | 14-09-1986 | GREEN C.H. |